# EUROPEAN PATENT SPECIFICATION

(11) **EP 4 506 747 B1**
(45) Date of publication and mention of the grant of the patent: **11.02.2026**
(21) Application number: 22936616.6
(22) Date of filing: 08.04.2022
(51) Int. Cl.: G02C 11/00, A61L 9/12, A61L 9/012, A61L 9/14, G02C 5/00, G02C 5/14

(54) **GLASSES CAPABLE OF SELECTIVE SMART CONTROL OF FRAGRANCE CAPSULE**
BRILLE MIT DER FÄHIGKEIT ZUR SELEKTIVEN INTELLIGENTEN STEUERUNG EINER DUFTKAPSEL
LUNETTES CAPABLES D'UNE COMMANDE INTELLIGENTE SÉLECTIVE D'UNE CAPSULE DE PARFUM

(43) Date of publication of application: 12.02.2025
(73) Proprietor: Lim, Seong Kyu, Daegu 41954 (KR); Lim, Jong Ho, Daegu 41954 (KR); Lim, Jong Yoon, Daegu 41954 (KR); Kim, Myeong Hee, Daegu 41954 (KR)
(72) Inventor: Lim, Seong Kyu, Daegu 41954 (KR); Lim, Jong Ho, Daegu 41954 (KR); Lim, Jong Yoon, Daegu 41954 (KR); Kim, Myeong Hee, Daegu 41954 (KR)
(74) Representative: Vossius & Partner Patentanwälte Rechtsanwälte mbB
(86) International application number: PCT/KR2022/005131
(87) International publication number: WO 2023/195562

(56) References cited:
- CN-B- 110 780 465
- CN-U- 212 647 185
- JP-U- 3 228 384
- JP-U- H 036 623
- KR-A- 20150 007 453
- KR-A- 20150 083 214
- KR-A- 20220 079 263
- KR-Y1- 200 364 065
- US-A1- 2005 162 610
- US-A1- 2018 071 425
- US-A1- 2019 196 191

## Description

### Technical Field

The present invention relates to glasses capable of selective smart control of a fragrance capsule and, more specifically, to a technology that applies a fragrance capsule in conjunction with a mechanical structure, selectively emitting fragrance using a smartphone, which is always carried by a user.

### Background Art

In general, glasses include a glass frame on which lenses are mounted and temples which are foldably attached on both sides of the frame.

Glasses are primarily worn by individuals with poor vision to aid in recognizing objects or text, thus making everyday life more comfortable. However, recently, many people wear glasses for sun protection, driving, sports, or fashion purposes regardless of vision correction.

Meanwhile, to enhance the functionality and commercial appeal of glasses, a lot of technologies that incorporate scent pads into the frame or the temples to emit fragrance around the wearer have been proposed.

First, conventional arts will be described.

Korean Patent Publication No. 10-2018-0106022 discloses a glass temple including: a temple body having a pad-receiving space, which is provided at the rear end and has an open top; a fragrance pad inserted into and removed from the pad-receiving space of the temple body; and a diffusion cap installed at the top of the pad-receiving space, and having a plurality of diffusion holes.

Korean Utility Model Registration No. 20-0364065 discloses a fragrance chip detachably attached to glasses, which includes a locking piece and an injection hole formed at one side so that the fragrance chip can be detachably attached to a temple, a storage space formed therein to be connected to the injection hole, and a fragrance-containing sponge inserted into the storage space.

The conventional arts relate to the technology of emitting fragrance to glasses and the fragrance chip detachably attached to glasses. However, the conventional arts can emit only fragrance, but have no fragrances specifically designed to repel insects such as mosquitoes.

Further relevant prior art comprises CN212647185U which discloses fragrance diffusing glasses, as well as JP3228384U which discloses spectacles which can diffuse a medical agent.

### Disclosure

### Technical Problem

Accordingly, the present invention has been made in view of the above-mentioned problems occurring in the related art, and it is an objective of the present invention to provide glasses capable of selective smart control of a fragrance capsule, which includes two or more fragrance capsules inserted into one side of a temple to selectively emit fragrance inside, can emit fragrance of the fragrance capsules solely or simultaneously, and can be controlled through a smart phone, thereby providing convenience in use. Meanwhile, the glasses capable of selective smart control of a fragrance capsule provides a structure into which the fragrance capsule can be inserted, thereby allowing a user to use the glasses according to use purposes.

### Technical Solution

To accomplish the above-mentioned objects, according to the present invention, there is provided a system comprising glasses capable of selective smart control of a fragrance capsule, which can emit fragrance and include temples respectively connected to both sides of a frame or lenses, including: a slide hole which is formed on one side of the temple; a fragrance capsule including a body part having a slide part formed at an outer side thereof to be slidably coupled to the slide hole and a capsule insertion groove formed at the upper center portion thereof and having a predetermined depth in the downward direction, a capsule containing a liquid having one or more fragrances therein and fitted to the capsule insertion groove, a pressing part inserted into the capsule insertion groove, positioned at the upper portion of the fragrance capsule and pressing one side of the capsule, a control part for controlling the pressing part selectively or periodically at predetermined time intervals, a battery part for supplying power to the control part, and communication means for communication with a smartphone; the system further comprising the smartphone in which an application for selectively controlling the control part is installed.

Two or more slide holes are formed on one side of the temple to be spaced apart from each other at a predetermined interval, two or more capsules containing the same fragrance or different fragrances are provided, and the application of the smartphone is configured to control the spraying of the capsules individually or simultaneously.

The capsule includes: a space part which, except a portion of an upper part, has flexible properties, and which is filled with a liquid fragrance; a hole which is formed on one side of an upper part thereof and extends from the space part to allow the fragrance-containing liquid to be filled or discharged; and a spray part which selectively seals the hole and is pressed by the pressing part to selectively discharge the fragrance-containing liquid.

### Advantageous Effect

According to an embodiment of this invention, the glasses capable of selective smart control of a fragrance capsule can maintain the inherent purpose of emitting fragrance, allow for easy replacement of the fragrance capsule through the selectively attachable and detachable structure formed on one side of the temple, and selectively adjust emission of fragrance of the fragrance capsule using a smartphone, thereby providing convenience.

### Description of Drawings

FIG. 1 is an enlarged perspective view illustrating glasses according to a preferred embodiment of the present invention.
FIG. 2 is a perspective view illustrating a fragrance capsule according to a preferred embodiment of the present invention.
FIG. 3 is an exploded view illustrating the fragrance capsule according to a preferred embodiment of the present invention.
FIG. 4 is a cross-sectional view illustrating the fragrance capsule according to a preferred embodiment of the present invention.
FIG. 5 is a view showing a usage state of the fragrance capsule according to a preferred embodiment of the present invention.
FIG. 6 is a view illustrating the fragrance capsule according to a preferred embodiment of the present invention.
FIG. 7 is a view illustrating a fragrance capsule according to another embodiment of the present invention.

200: Temple 210: Slide hole
300: Fragrance capsule
310: Body part 311: Slide part 313: Capsule insertion groove
320: Capsule 321: Space part 323: Hole
325: Spray part
330: Pressing part 340: Control part 350: Battery part
360: Communication means 400 : Smartphone

### Mode for Invention

The present invention provides glasses capable of selective smart control of a fragrance capsule, which apply a fragrance capsule like a mechanical structure and can selectively emit fragrance using a smartphone, which is always carried by a user.

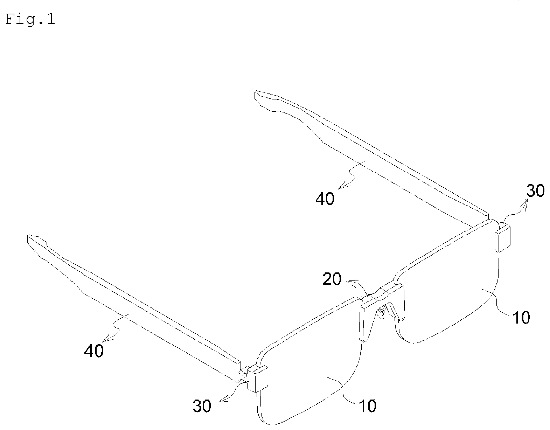

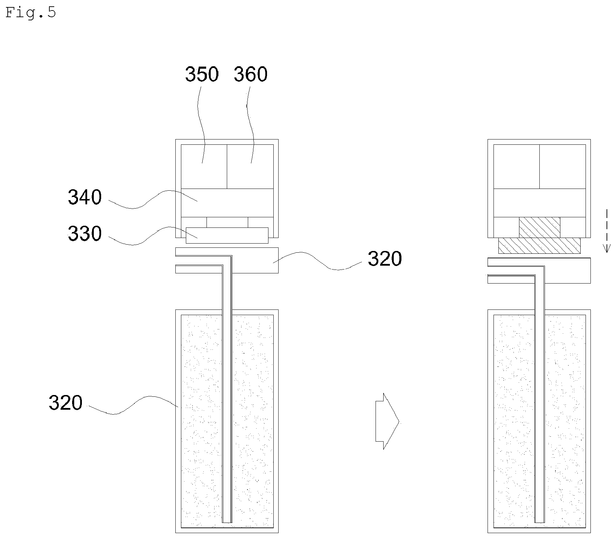

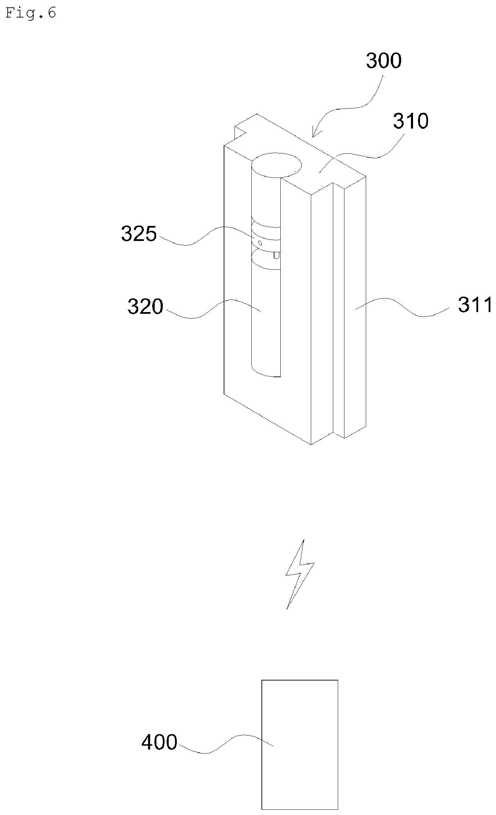

Hereinafter, with reference to the accompanying drawings, the preferred structure and operation of the present invention for achieving the objectives of the present invention will be described with reference to FIGS. 1 to 7.

The configuration of the present invention will be described.

The system comprising glasses capable of emitting fragrance, which include temples 200 respectively connected to both sides of a frame or lenses, comprise: a slide hole 210 which is formed on one side of the temple 200; a fragrance capsule 300 including a body part 310 having a slide part 311 formed at an outer side thereof to be slidably coupled to the slide hole 210 and a capsule insertion groove 313 formed at the upper center portion thereof and having a predetermined depth in the downward direction, a capsule 320 containing a liquid having one or more fragrances therein and fitted to the capsule insertion groove 313, a pressing part 330 inserted into the capsule insertion groove 313, positioned at the upper portion of the fragrance capsule 300 and pressing one side of the capsule 320, a control part 340 for controlling the pressing part 330 selectively or periodically at predetermined time intervals, a battery part 350 for supplying power to the control part 340, and a communication means 360 for communication with a smartphone 400; the system further comprising the smartphone 400 in which an application for selectively controlling the control part 340 is installed.

That is, the control module 400 configured in the smartphone 400, which users always carry, and the temple 200 can discharge the liquid containing fragrance inside the capsule 320 to the outside.

At this time, the fragrance may include various scents, and if necessary, may include scents that can repel insects such as mosquitoes and flies.

The glasses of the present invention can substantially include a glass frame, or can be used by connecting the temples 200 to lenses without the glass frame.

Meanwhile, if the glasses include the glass frame, the glass frame can have a full-rim or a half-rim, and if the glasses do not include the glass frame, the lenses can be used with various changes in size and shape.

In the present invention, as an embodiment, the glasses include a full-rim frame, but the present invention is not limited thereto.

The temple 200 has a common configuration that one side of the temple 200 is hinge-coupled to an end piece of the frame to pivot around the hinge to one side or the other side, and the other side of the temple 200 connected to the hinge extends inclinedly downward to be retained to a user's ear, thus preventing the glasses from slipping off when worn.

Here, the temple 200 of the present invention includes, as one of the key components, the slide hole 210 which is formed vertically at one side of an upper portion thereof as illustrated in FIG. 1 and has a predetermined depth. The slide hole 210 is formed in a "T" shape when viewed from the plane to correspond to the external appearance of the slide part 311 of the temple 200, thus allowing a vertical fitting and preventing horizontal separation.

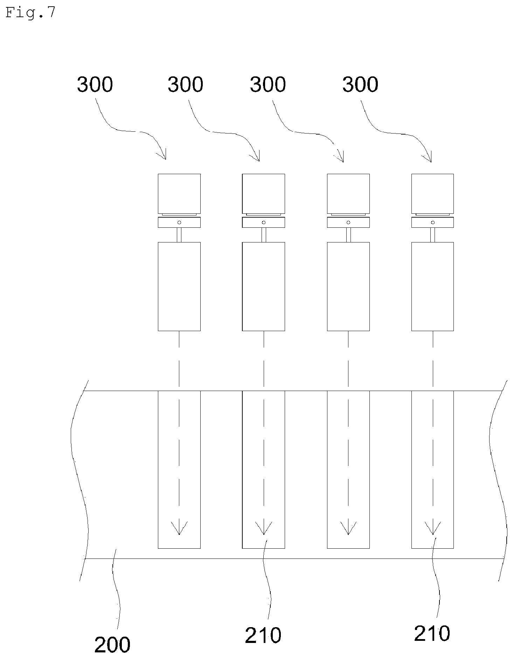

Additionally, the slide hole 210, as illustrated in FIG. 7, may have two or more protrusions formed on both sides to enable a more robust fixation.

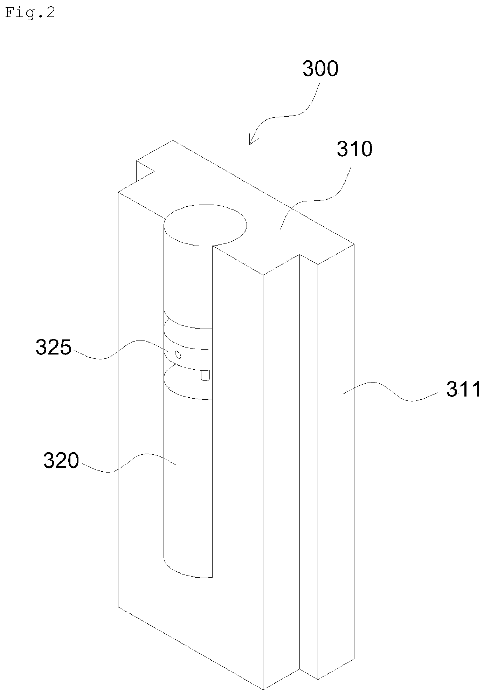

Referring to FIGS. 2 to 5, the fragrance capsule 300 includes the body part 310 and the capsule 320.

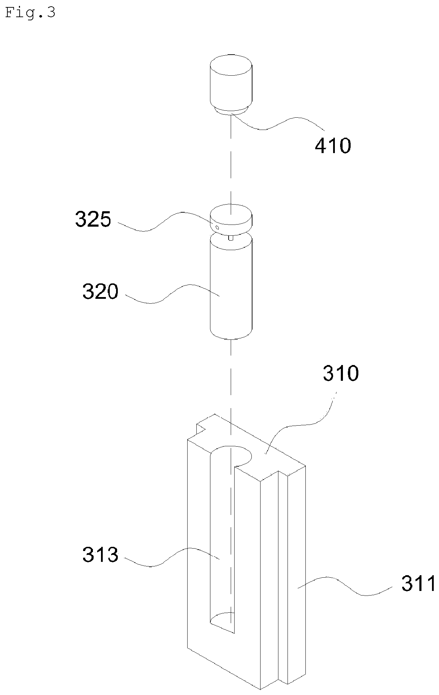

The body part 310, as illustrated in FIGS. 2 or 3, has the slide part 311 slidingly coupled with the slide hole 210, that is, the slide part 311 is also formed in a "T" shape to fit into the slide hole 210, and the capsule insertion groove 313 is formed in the upper central part where the capsule 320 will be inserted.

Meanwhile, the capsule 320 is designed to contain the liquid that emits fragrance. In this case, the term "liquid" refers to any liquid that emits fragrance, including those that emit various scents, such as scents that insects dislike, sweet scents, and other fragrances.

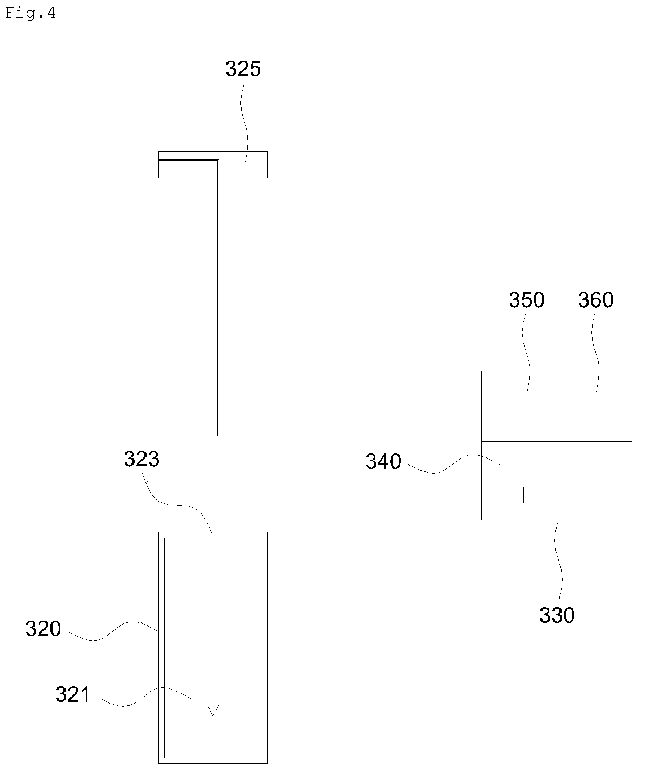

Here, as illustrated in FIGS. 3 or 4, the capsule 320, except for a portion of an upper part, has flexible properties, and includes the space part 321 that can be filled with liquid fragrance, the hole 323 formed on the upper side and extending from the space part 321 to fill or discharge the fragrance-containing liquid, and the spray part 325 which is designed to selectively seal the hole 323 or selectively discharge the fragrance-containing liquid by being pressed by the pressing part 330.

That is, the capsule 320 has the flexible properties to prevent damage to the capsule 320 due to impacts or similar causes. The flexible configuration of the capsule 320 excluding a portion of the upper part is preferable because a relatively hard part is necessary to maintain a fixed form when the spray part 325 is pressed by the pressing part 410.

In the drawings, the capsule 320 is not divided into a flexible part and a non-flexible part.

Meanwhile, as illustrated in FIG. 4, the space part 321, as previously described, is generally intended for fragrance spraying, allowing the fragrance-containing liquid to be sprayed so that the fragrance contained in the liquid can be emitted. That is, the space part is a space designed to hold such a liquid.

Additionally, although not illustrated, the space can be formed as a single entity or divided into multiple parts as needed, so that liquids with the same or different fragrances can be filled in the formed space part 321.

In this instance, when the space part 321 is formed into multiple parts and each space part is filled with different fragrances, the spray part 325 can be individually connected to each space part 321 or a single spray part 325 can be used to spray the liquid from each space part 321.

The hole 323 serves as a passage to fill or spray the fragrance-containing liquid into the space part 321, and can be formed as one or in multiple.

As illustrated in FIG. 4, the spray part 325 is formed on the non-flexible part of the top of the capsule 320 and is designed to spray the fragrance-containing liquid from the space part 321 with a single press.

In this instance, the spray part 325 can have a conventional pump structure, and if necessary, can have a structure that can selectively spray liquid outward.

In addition, the spray part 325 is formed corresponding to the number of space parts 321 and the number of holes 323.

For example, if multiple space parts 321 are formed and each has a hole 323, each spray part 325 can be configured above each hole 323 so as to spray the liquid contained in any one of the space parts 321 in accordance with the operation of the pressing part 330.

If multiple space parts 321 are formed without any hole 323 for each and only one hole 323 is formed at the uppermost side, only one spray part 325 can be configured at the hole 323.

Such a structure allows users to purchase and use a capsule 320 with a structure that suits their usage purpose and needs.

Furthermore, the capsule 320 can ultimately emit a single fragrance or a mixed fragrance depending on whether the liquid positioned in the multiple space part 321 contains the same fragrance or different fragrances.

When multiple space parts 321 include liquids containing different fragrances and one hole 323 and one spray part 325 are configured, as soon as the spray part 325 is pressed, different fragrances contained in the multiple space parts 321 are sprayed from the spray part 325 at one, thus allowing multiple fragrances to be mixed.

Additionally, if the spray part 325 has a plurality of suction parts, the liquid of each space part 321 is not mixed before sprayed and the fragrances of the liquid can be mixed after sprayed. If the spray part 325 has a single spraying part and suction parts corresponding to the number of the space parts 321, fragrances are mixed before sprayed by the spray part 325, so the mixed fragrances can be discharged.

Meanwhile, to selectively control the capsule 320, the pressing part 330, the control part 340, the battery part 350, and the communication means 360 are substantially fit into the capsule insertion groove 313 in a modular form and arranged above the capsule 320. That is, the fragrance capsule includes: the pressing part 330 positioned to be in contact with the spray part 325 to spray the fragrance-containing liquid; the control part 340 located above the pressing part 330 to receive control commands from the smartphone 400 and control the operation of the pressing part 330; the battery part 350 located above the control part 340 to supply power to the control part 340; and the communication means 360 for operating the control part 340 by receiving signals from the smartphone 400.

As illustrated in FIGS. 3 to 5, the pressing part 330 is cylindrical and moves in one direction according to the control commands from the control part 340 to press the spray part 325 closely contacting the pressing part 330, enabling the liquid to be sprayed.

Here, the pressing part 330 can apply a mechanical structure or an electromagnetic structure, and is preferably designed to return to its original state after being pressed.

The control part 340 typically controls any mechanical or electronic configuration, and the battery part 350 and the communication module 360 are of conventional configuration, further detailed description thereof will be omitted.

In this instance, the control part 340 can operate the pressing part 330 selectively or periodically like a timer.

As illustrated in FIG. 7, two or more slide holes 210 are formed to be spaced apart from each other on one side of the temple 200, and two or more capsules 320 with the same or different fragrances are provided, and the spray of the capsule 320 can be controlled solely or simultaneously by an application of the smartphone 400.

The space part 321 formed in the capsule 320 are not formed to be divided but the plurality of capsules 320 are formed, so that each capsule 320 can be selectively controlled by the smartphone 400.

According to an embodiment of this invention, the glasses capable of selective smart control of the fragrance capsule can maintain the inherent purpose of emitting fragrance, allow for easy replacement of the fragrance capsule through the selectively attachable and detachable structure formed on one side of the temple, and selectively adjust emission of fragrance of the fragrance capsule using a smartphone, thereby providing convenience.

## Claims

1. A system comprising glasses capable of selective smart control of a fragrance capsule, which can emit fragrance and include temples (200) respectively connected to both sides of a frame or lenses, comprising:
a slide hole (210) which is formed on one side of the temple (200);
a fragrance capsule (300) including a body part (310) having a slide part (311) formed at an outer side thereof to be slidably coupled to the slide hole (210) and a capsule insertion groove (313) formed at the upper center portion thereof and having a predetermined depth in the downward direction, a capsule (320) containing a liquid having one or more fragrances therein and fitted to the capsule insertion groove (313), a pressing part (330) inserted into the capsule insertion groove (313), positioned at the upper portion of the fragrance capsule (300) and pressing one side of the capsule (320), a control part (340) for controlling the pressing part (330) selectively or periodically at predetermined time intervals, a battery part (350) for supplying power to the control part (340), and communication means (360) for communication with a smartphone (400); and
the system further comprising the smartphone (400) in which an application for selectively controlling the control part (340) is installed.

2. The system according to claim 1, wherein two or more slide holes (210) are formed on one side of the temple (200) to be spaced apart from each other at a predetermined interval,
wherein two or more capsules (320) containing the same fragrance or different fragrances are provided, and
wherein the application of the smartphone (400) is configured to control the spraying of the capsules (320) individually or simultaneously.

3. The system according to claim 1, wherein the capsule (320) includes:
a space part (321) which, except a portion of an upper part, has flexible properties, and which is filled with a liquid fragrance;
a hole (323) which is formed on one side of an upper part thereof and extends from the space part (321) to allow the fragrance-containing liquid to be filled or discharged; and
a spray part (325) which selectively seals the hole (323) and is pressed by the pressing part (330) to selectively discharge the fragrance-containing liquid.

## Patentansprüche

1. System mit einer Brille, die zu selektiver intelligenter Steuerung einer Duftstoffkapsel, die Duftstoff abgeben kann, fähig ist und Bügel (200) beinhaltet, die jeweils mit einer von zwei Seiten eines Rahmens oder Gläsern verbunden sind, aufweisend:
ein Gleitloch (210), das auf einer Seite des Bügels (200) gebildet ist;
eine Duftstoffkapsel (300), die einen Körperteil (310) mit einem an einer äußeren Seite desselben gebildeten Gleitteil (311), um gleitfähig an das Gleitloch (210) gekoppelt zu werden, und einer Kapseleinstecknut (313), die an dem oberen mittleren Abschnitt desselben gebildet ist und eine vorgegebene Tiefe in der Abwärtsrichtung aufweist, eine Kapsel (320), die eine Flüssigkeit mit einem oder mehreren Duftstoffen darin enthält und in der Kapseleinstecknut (313) montiert ist, einen in die Kapseleinstecknut (313) eingesteckten Druckteil (330), der an dem oberen Abschnitt der Duftstoffkapsel (300) positioniert ist und eine Seite der Kapsel (320) drückt, einen Steuerteil (340) zum Steuern des Druckteils (330) selektiv oder periodisch in vorgegebenen Zeitabständen, einen Batterieteil (350) zum Liefern von Leistung an den Steuerteil (340) und Kommunikationsmittel (360) aufweist zur Kommunikation mit einem Smartphone (400); und
wobei das System ferner das Smartphone (400) umfasst, in dem eine Anwendung zum selektiven Steuern des Steuerteils (340) installiert ist.

2. System nach Anspruch 1, wobei zwei oder mehr Gleitlöcher (210) auf einer Seite des Bügels (200) gebildet sind, um in einem vorgegebenen Abstand voneinander beabstandet zu sein,
wobei zwei oder mehr den gleichen Duftstoff oder unterschiedliche Duftstoffe enthaltende Kapseln (320) bereitgestellt sind und
wobei die Anwendung des Smartphones (400) dazu konfiguriert ist, das Sprühen der Kapseln (320) einzeln oder gleichzeitig zu steuern.

3. System nach Anspruch 1, wobei die Kapsel (320) aufweist:
einen Raumteil (321), der mit Ausnahme eines oberen Teils biegsame Eigenschaften aufweist und der mit einem flüssigen Duftstoff gefüllt ist;
ein Loch (323), das auf einer Seite eines oberen Teils davon gebildet ist und sich von dem Raumteil (321) erstreckt, um zu ermöglichen, dass die Duftstoff enthaltende Flüssigkeit eingefüllt oder ausgestoßen wird; und
einen Sprühteil (325), der das Loch (323) selektiv verschließt und von dem Druckteil (330) gedrückt wird, um die Duftstoff enthaltende Flüssigkeit selektiv auszustoßen.

## Revendications

1. Système comprenant des lunettes capables d'une commande intelligente sélective d'une capsule de parfum, qui peut dégager un parfum et comprendre des branches (200) respectivement reliées aux deux côtés d'une monture ou de verres, comprenant :
un trou de coulissement (210) qui est formé sur un côté de la branche (200) ;
une capsule de parfum (300) comprenant une partie corps (310) présentant une partie coulissante (311) formée au niveau d'un côté externe de celle-ci pour être accouplée de manière coulissante au trou de coulissement (210) et une rainure d'insertion de capsule (313) formée au niveau de la partie centrale supérieure de celle-ci et présentant une profondeur prédéterminée dans la direction vers le bas, une capsule (320) contenant un liquide ayant un ou plusieurs parfums en son sein et ajustée sur la rainure d'insertion de capsule (313), une partie de pression (330) insérée dans la rainure d'insertion de capsule (313), positionnée au niveau de la partie supérieure de la capsule de parfum (300) et pressant un côté de la capsule (320), une partie de commande (340) permettant de commander la partie de pression (330) sélectivement ou périodiquement à des intervalles de temps prédéterminés, une partie batterie (350) permettant d'alimenter la partie de commande (340), et des moyens de communication (360) pour la communication avec un téléphone intelligent (400) ; et
le système comprenant en outre le téléphone intelligent (400) dans lequel une application permettant de commander sélectivement la partie de commande (340) est installée.

2. Système selon la revendication 1, dans lequel deux ou plus de deux trous de coulissement (210) sont formés sur un côté de la branche (200) pour être espacés l'un de l'autre selon un intervalle prédéterminé,
dans lequel deux ou plus de deux capsules (320) contenant le même parfum ou différents parfums sont fournies, et
dans lequel l'application du téléphone intelligent (400) est configurée pour commander la pulvérisation des capsules (320) individuellement ou simultanément.

3. Système selon la revendication 1, dans lequel la capsule (320) comprend :
une partie d'espace (321) qui, à l'exception d'une portion d'une partie d'espace, présente des propriétés flexibles, et qui est remplie d'un parfum liquide ;
un trou (323) qui est formé sur un côté d'une partie supérieure de celle-ci et s'étend à partir de la partie d'espace (321) pour permettre au liquide contenant un parfum d'être rempli ou évacué ; et
une partie de pulvérisation (325) qui assure sélectivement l'étanchéité du trou (323) et est pressée par la partie de pression (330) pour évacuer sélectivement le liquide contenant un parfum.
